# EUROPEAN PATENT SPECIFICATION

(11) **EP 1 912 661 B1**
(45) Date of publication and mention of the grant of the patent: **16.03.2011**
(21) Application number: 05809011.9
(22) Date of filing: 23.09.2005
(51) Int. Cl.: A61L 27/38, A61L 27/50, A61K 38/36, A61K 38/39

(54) **SIMPLE METHOD OF TRANSPLANTING INJECTABLE CHONDROCYTE FOR AUTOLOGOUS CHONDROCYTE TRANSPLANTATION**
EINFACHES VERFAHREN ZUR TRANSPLANTATION VON INJIZIERBAREN CHONDROZYTEN FÜR EINE AUTOLOGE CHONDROZYTEN-TRANSPLANTATION
METHODE SIMPLE DE TRANSPLANTATION D'UN CHONDROCYTE INJECTABLE POUR UNE TRANSPLANTATION DE CHONDROCYTE AUTOLOGUE

(30) Priority: 20.07.2005 KR 20050066026
(43) Date of publication of application: 23.04.2008
(73) Proprietor: Sewon Cellontech Co., Ltd., Youngdeungpo-Gu, Seoul 150-712 (KR)
(72) Inventor: KO, Chang-Kwon, Seoul 139-921 (KR); LEE, Eun-Young, Seoul 140-190 (KR); CHOI, Jeong-Yong, Gyeonggi-do 437-070 (KR); JANG, Jae-Deog, Seoul 139-784 (KR); CHANG, Cheong-Ho, Seoul 137-040 (KR); KIM, Pyoung-Min, Seoul 133-112 (KR)
(74) Representative: Merkle, Gebhard
(86) International application number: PCT/KR2005/003147
(87) International publication number: WO 2007/011094

(56) References cited:
- EP-A- 1 374 857
- EP-A- 1 535 633
- EP-A2- 1 509 118
- WO-A1-2005/060987
- WO-A2-00/09179
- US-A- 5 842 477
- US-A1- 2001 014 475
- US-A1- 2002 082 220

## Description

### Technical Field

The present invention relates to a cartilage therapeutic composition for use in a method for transplanting a cartilage therapeutic(Chondron: in vitro cultured chondrocytes for autologous chondrocyte transplantation) via injection. More specifically, the present invention relates to a cartilage therapeutic composition for use in a method for transplanting a cartilage therapeutic involving mixing and transplanting matrices including fibrin, hyaluronic acid and collagen, constituting main ingredients of cartilage, by which inconvenience of conventional osteoperiosteal grafting is solved, treatment of a wide range of defects and worsened osteoarthritis is possible and burden of patients to be treated is alleviated via a simplified surgical operation method, thus more rapidly and effectively promoting generation of cartilage and improving consumer satisfaction.

### Background Art

Recently, molecular biology, cell biology, developmental biology and tissue engineering, as well known, have contributed to improvements in health and quality of life via development of methods of improving, maintaining, and restoring function to damaged tissues and organs.

Tissue engineering produces tissue substitutes by controlling cell adhesion, growth and differentiation, and activity of cytokines and growth factors via interaction with a cell-based matrix as a tissue replacement to thereby secrete intracellular signaling substances. Early work in tissue engineering has primarily focused on utilization of cells alone or cell therapy utilizing a matrix complex produced by cells, thus greatly limiting applications thereof leading to an urgent need for the development of more advanced tissue substitutes, and therefore has led to development of cartilage therapeutics utilizing chondrocytes and matrix mixtures.

It has been demonstrated that instability of joints and partial deficiency of menisci accelerate abrasion and damage of articular surfaces. In addition, articular cartilage, once damaged, cannot be fully regenerated in vivo. Chronic stimulation applied to the damaged articular cartilage causes articular pain and progressive limitation of articular movement, resulting in fatal degenerative arthritis. Chondrocytes that are non-proliferable in vivo can be effectively used in regeneration of joints by inducing differentiation and proliferation of chondrocytes via adjustment of culture conditions in vitro. As a result, transplanted chondrocytes acquire histological and mechanical properties similar to those of cartilage of normal joints, and thus transplantation rejection or tissue destruction is not observed with good results in more than 90% of patients.

Meanwhile, autologous chondrocyte transplantation (ACT) is an effective therapy for treatment of damaged articular cartilage. Other therapies such as abrasion arthroplasty, drilling and microfracture, autograft of periosteum and perichondrium involve replacement of damaged articular cartilage with fibrocartilage or fibrocartilaginous tissues with no substantial production of hyaline cartilage. However, as compared to any other conventional techniques, the most important advantage of ACT consists in production of hyaline cartilage.

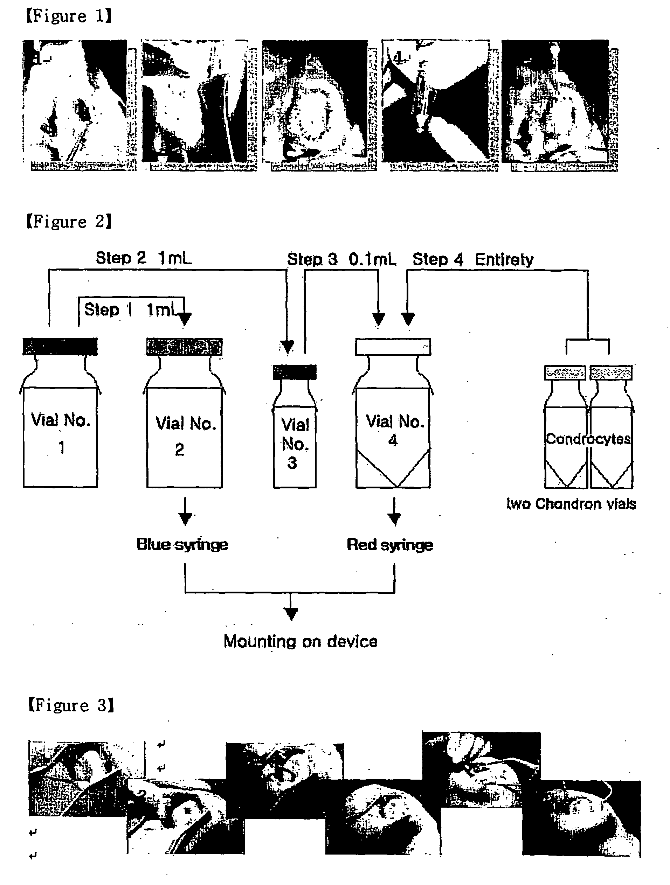

In spite of such an excellent advantage, autologous chondrocyte transplantation (ACT) suffers from some technical and theoretical difficulties as shown in Fig. 1.

That is, firstly, this autologous chondrocyte transplantation technique requires a large incision of the surgical site for collection of periosteum. Such a large incision results in severe pain following surgery and reduced knee motility.

Secondly, suturing of the periosteal patch on the boundary of a cartilage defect area requires a very complicated surgical technique which has been reported to sometimes accompany periosteal hypertrophy and intra-articular adhesion. Suturing of periosteum is the most time-consuming process among surgical processes for conducting autologous chondrocyte transplantation.

Additionally, autologous chondrocyte transplantation is disadvantageous in that leakage of chondrocytes from the transplanted sites sometimes occurs during the rehabilitation process such as therapeutic exercise of the knees after surgical operation, and the transplanted chondrocytes, which are present in a liquid phase in the transplanted sites, are localized toward one side by action of gravity, thus resulting in uneven distribution and heterogeneous growth of cartilage.

In order to solve the above-mentioned problems, a great deal of research has focused on the utilization of biodegradable and biocompatible polymers as a carrier or scaffold. For example, a great deal of research into tissue engineering for cartilage in vivo and in vitro has been actively undertaking, utilizing naturally-occurring polymers such as collagen, hyaluronic acid and fibrin as biomaterials, or utilizing synthetic polymers such as polylactic acid or polyglycolic acid.

The scaffold plays an important role in maintenance of a pore structure necessary for growth and proliferation of cells and phenotypes of chondrocytes. Fibrin and hyaluronic acid are suitable polymers satisfying the requirements for use as the scaffold, as mentioned above. Fibrin is a naturally-occurring substance and is a very suitable biological carrier for chondrocyte transplantation. In addition, fibrin has already been reported to have advantages such as biocompatibility, biodegradability and capability to bind to subchondral bones (bones that are structurally adjacent to, that is, beneath or below the cartilage). Furthermore, Fibrin structures can be easily modified into desired shapes and is readily used for injection. Meanwhile, hyaluronic acid is a lubricating agent, found in the synovial joint fluid, and serves to control content of moisture in joints.

US 2001/0014475 A1 discloses a method for growing cells on biodegradable microcarrier particles and more specifically growing chondrocytes for an extended period of time until they aggregate. These aggregated cells can be injected directly or shaped for implantation into the body.

US 2002/0082220 A1 relates to a method and polymer composition for repairing a tissue of a patient wherein the polymer composition preferably comprises at least one blood component. The blood component may also comprise fibrinogen and thrombin

WO 2005/060987 A1 relates to a cartilage therapeutic composition for repairing damaged cartilage tissue. It comprises a mixture of components of chondrocytes, and thrombin and a fibrinogen matrix containing fibrinogen.

However, one problem still remains. Since most scaffolds for tissue engineering are solid types such as patches, sponges and sheets, cartilage therapeutics for tissue engineering are difficult to bind to adjacent native cartilage. This fact is of importance in that cartilage therapeutics transplanted into cartilage defect regions exerts incomplete cure of the boundary region therebetween.

### Disclosure of Invention

### Technical Problem

The present invention has been made in view of the above problems, and provides a cartilage therapeutic composition for use in a method for transplantation of a cartilage therapeutic via injection.

Therefore, it is a first object of the present invention to solve inconvenience of conventional osteoperiosteal grafting by mixing and transplanting matrices including collagen, hyaluronic acid and fibrin which are main ingredients of cartilage.

A second object of the present invention is to provide convenient in situ transplantation via utilization of rapid gelling characteristics in which an injectable cartilage therapeutic is gelated within a short period of time, i.e., about 1 to 5 minutes, upon transplanting.

A third object of the present invention is to enable transplanted sites to be completely bound to the adjacent native cartilages, as well as to enable treatment of a wide variety of cartilage defects and severe osteoarthritis.

A fourth object of the present invention is to alleviate burden of patients to be treated via a simplified surgical operation method.

A fifth object of the present invention is to more rapidly and effectively promote generation of cartilage, thereby enhancing customer satisfaction via such a simplified surgical operation method.

### Technical Solution

There is provided a cartilage therapeutic composition for use in a method of transplanting an injectable chondrocyte for autologous chondrocyte transplantation, comprising;
placing 1 ml of a chondrocyte culture contained in Vial No. I (Red) into a 1 ml sterile syringe and injecting the cell culture into Vial No. 2 (Blue) containing white or light-yellow lyophilized powder (fibrinogen) by inserting the syringe into Vial No. 2 in the vertical direction;
injecting I ml of the cell culture contained in Vial No. I (Red) into Vial No. 3 (Red, small) containing white lyophilized powder (thrombin) by inserting the syringe into Vial No. 3 in the vertical direction, thereby gradually injecting red liquid;
collecting 0.1 cc of I ml content of Vial No. 3 (Red, small) using a 1 ml syringe, followed by injection into a bottom of Vial No. 4 (Yellow);
adding the entire contents of two vials containing chondrocyte suspension to Vial No. 4 (Yellow) using a 1 mL sterile syringe, followed by mixing the contents two or three times using the syringe;
confirming complete dissolution of the contents of Vial No. 2 (Blue) and then injecting the entirety of the dissolved material into a 1 ml syringe;
injecting the well-mixed cell therapeutic of Vial 4 (Yellow) into a 1 ml syringe; and
mounting two 1 ml syringes filled with the contents of Vial No. 2 (Blue) and Vial No. 4 (Yellow) on a standing holder followed by mounting a screw tip on the syringes, and mixing the contents of the syringes using the front end mixing tip thereof while injecting the resulting mixture to be transplanted into a damaged cartilage region of an animal.

### Brief Description of the Drawings

The above and other objects, features and other advantages of the present invention will be more clearly understood from the following detailed description taken in conjunction with the accompanying drawings, in which:
Fig. 1 is a photograph showing a conventional method of transplanting a cartilage therapeutic;
Fig. 2 is a photograph showing a method of transplanting an injectable cartilage therapeutic, which is applied to the present invention;
Fig. 3 is a photograph showing a method of transplanting a cartilage therapeutic, which is applied to the present invention; .
Fig. 4 is a photograph showing a method of transplanting an injectable cartilage therapeutic, which is applied to the present invention, via use of a syringe;
Fig. 5 is a photograph showing physical properties of an injectable cartilage therapeutic in accordance with the present invention;
Fig. 6 is a photograph taken 3 months after regeneration of canine cartilage in accordance with an example of the present invention;
Fig. 7 is an EM taken 8 weeks after transplantation of a cartilage therapeutic in accordance with the present invention;
Fig. 8 is an EM taken 12 weeks after transplantation of a cartilage therapeutic in accordance with the present invention;
Fig. 9 is a photograph taken 12 weeks after regeneration of a canine cartilage tissue in accordance with an example of the present invention;
Fig. 10 is a photograph showing the results of histological examination of a cartilage therapeutic in accordance with the present invention; and
Fig. 11 is a photograph showing the results of immunohistological examination of a cartilage therapeutic in accordance with the present invention.

### Best Mode for Carrying Out the Invention

The preferred embodiments of the present invention will now be described in more detail with reference to the accompanying drawings.

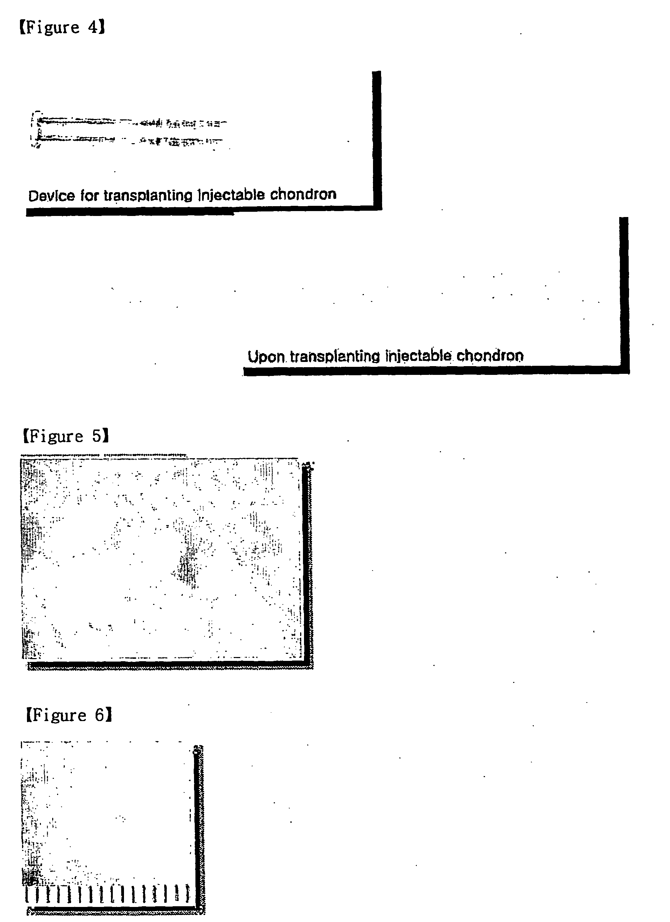

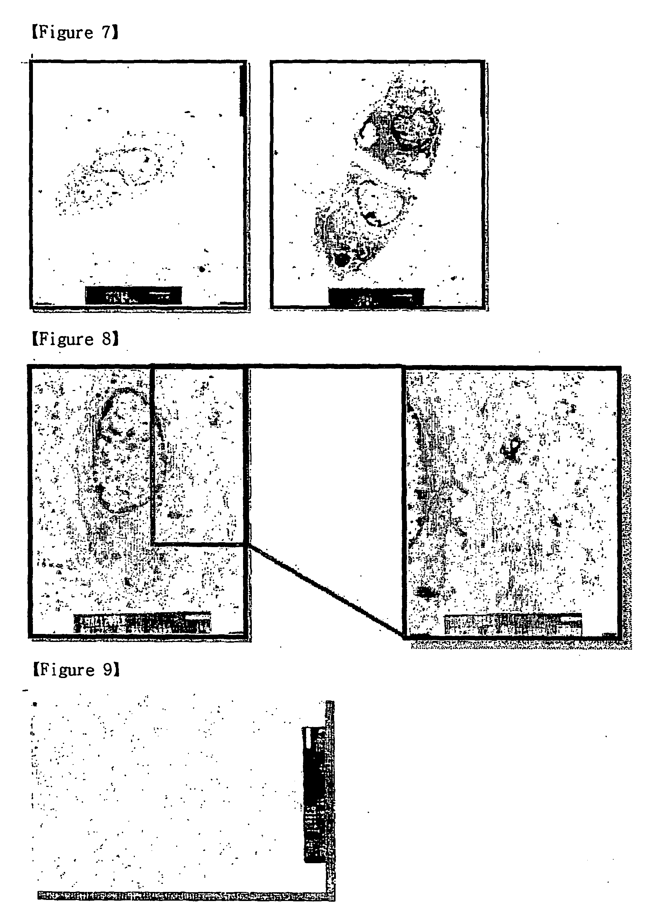

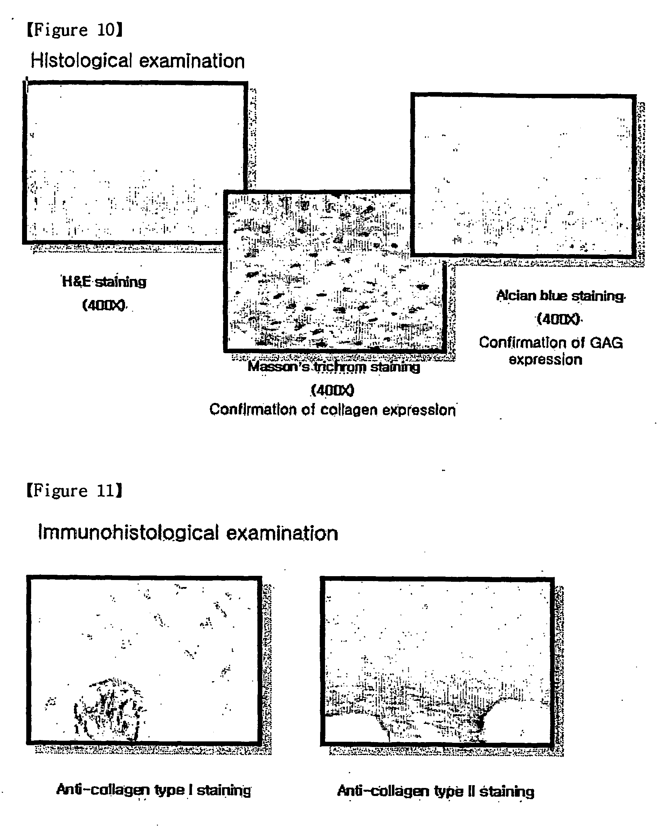

A method for transplantation of a cartilage therapeutic via injection, which is applied to the present invention, is constituted as shown in Figs. 2 through 12.

In connection with description of the present invention hereinafter, if it is considered that concrete description of known functions or constructions related to the present invention may make the subject of the present invention unclear, the detailed description thereof will be omitted.

Terms which will be described hereinafter are established taking into consideration functions in the present invention and may vary according to the custom or manufacturer s intention. Therefore, the terms used herein should be defined based on the contents throughout the specification of the present invention.

The present invention is characterized by mixing matrices including fibrin, hyaluronic acid and collagen, constituting main ingredients of animal cartilage, via use of a front end mixing tip of a syringe, while injecting the resulting mixture into a damaged cartilage region.

That is, the present invention enables the following: convenient in situ transplantation due to rapid gelling characteristics in which an injectable cartilage therapeutic is gelated within a short period of about 1 to 5 minutes, upon transplanting; complete binding of transplanted sites to the adjacent native cartilage, as well as treatment of a wide variety of cartilage defects and severe osteoarthritis; alleviation of burden applied to patients via a simplified surgical operation method; and thereby promotion of rapider and more effective generation of cartilage.

The present invention provides a cartilage therapeutic composition for use in a method for transplanting a cartilage therapeutic of the present invention via injection, according to the following example.

### EXAMPLE

In this example, experiments using animal (canine) chondrocytes were carried out . according to the-following sequence. Experimental results were evaluated 3 months later. That is, as shown in Fig. 2,
1. The limbus of cartilage defect region is trimmed and the size thereof is measured. Then, the following steps are carried out.
2. Among the contents in a kit, an aluminum cap of Vial No. 1 (Red) is removed and the cap and surface of the vial are washed with 70% ethanol. Herein, Vial No. 1 (Red) contains a cultured cell suspension of reddish color (chondrocyte culture + hyaluronic acid + aprotinin, etc.).
3. An aluminum cap of Vial No. 2 (Blue) is removed and the cap and surface of the vial are washed with 70% ethanol. Herein, Vial No. 2 (Blue) is a colorless vial containing a white or light yellow lyophilized powder (fibrinogen).
4. 1 ml of the content in Vial No. 1 (Red) is introduced into a 1 ml sterile syringe and the syringe needle is inserted into Vial No. 2 (Blue) which is stood vertically. Herein, the content of the syringe are directly injected over the lyophilizate, not injecting along the inner wall of the vial, while carefully preventing a needle tip from contacting the lyophilizate. In this connection, Vial No. 2 (Blue) is gently shaken with hands until the lyophilizate in Vial No. 2 (Blue) is completely dissolved.
5. An aluminum cap of Vial No. 3 (Red, small) is removed and the cap and surface of the vial are washed with 70% ethanol. Herein, Vial No. 3 (Red, small) is a colorless vial containing a white lyophilized powder (thrombin).
6. 1 ml of a solution contained in Vial No. 1 (Red) is introduced into a syringe and the syringe needle is inserted into Vial No. 3 (Red, small) which is stood vertically, thereby gradually injecting red liquid.
7. After removing an aluminum cap of Vial No. 4 (Yellow) and washing the cap and surface of the vial with 70% ethanol, 0.1 cc of I ml content in Vial No. 3 (Red, small) is collected using a I ml syringe and is injected to a bottom of Vial No. 4 (Yellow).
8. The entire contents of two vials containing chondrocyte suspension are introduced into Vial No. 4 (Yellow) using a 1 ml sterile syringe, followed by mixing two or three times using the syringe.
9. After confirming complete dissolution of the content of Vial 2 (Blue), the entirety of the dissolved material is filled into a 1 ml syringe.
10. The well-mixed cell therapeutic of Vial 4 (Yellow) is introduced into a 1 ml syringe.
11. Two I ml syringes filled with the contents of Vial No. 2 (Blue) and Vial No. 4 (Yellow), respectively, are mounted on a syringe standing holder.
12. A screw connecting tip is mounted on the syringes, followed by mounting a screw tip and a piston support.
13. After completion of assembly, transplantation is initiated. Herein, if possible, injection of the cartilage therapeutic is carried out at a time, without stopping.
14. About 5 minutes after completion of transplantation, the limbus of the transplantation site is trimmed.

A comparison between the present invention and conventional autologous chondrocyte transplantation (ACT) is shown in Table 1 below.

**Table 1**

| Conventional ACT | Inventive ACT (Injectable Chondron) |
|---|---|
| Liquid injectable preparation | Gel type (convenient transplantation due to rapid gelling characteristics within I to 5 minutes, upon transplanting, and readiness to form desired shapes |
| Chondrocytes + culture medium | Chondrocytes + biocompatible materials |
| Osteoperiosteal grafting necessary | Periosteum unnecessary (transplantable via minimal incision) |
| Excellent cartilage regeneration ability | More advanced cartilage regeneration ability |
| Knee incision necessary (incision of more than 15 cm necessary for suture, upon periosteum collection and transplantation) | Capable of performing surgery via minimal incision (5 cm),Capable of performing surgeryusing an arthroscope |
| Non-even distribution (possibility of unequal and localized cartilage regeneration by action of gravity, due to suspending state of cells in a liquid phase,) | Even distribution (exhibiting even distribution due to rapid gelling characteristics within a period of 1 to 5 minutes at a moment of transplantation and thereby homogeneous cartilage generation ability at that state) |
| Suture (the most time-consuming process among surgical processes and being complicated) | No suture |
| Long Operation time | Short Operation time |
| Producing fibrocartilage by bleeding | Bleeding control with bone wax (mostly producing hyaline cartilage) |
| | Addition of column generating processes (see cartilage therapeutic composition and use thereof) |
| | Transplantable regardless of defect shapes |
| | Alleviation of cell leakage due to incomplete suture |
| | Reducing the time for weight-bearing due to no periosteum suture |

Meanwhile, as the medium for cell culture that can be used in the present invention, mention may be made of DMEM (Dulbecco's Modified Eagle's Medium), Ham's F-12, DMEM/F-12, IMDM (Iscove's Modified Dulbecco's Medium), McCoy's 5A Medium, MEM (Minimum Essential Medium), alpha-MEM, RPM1 1640, Leibovitz's L-15 Medium and Eagle's basal Medium, which are conventionally used in the art.

Preferably, the concentration of the lyophilized powder (fibrinogen) used herein is in the range of 10 mg/mL to 200 mg/mL. Where the concentration of fibrinogen is less than 10 mg/mL, it is too low to exert properties thereof as the matrix. In contrast, where the concentration of fibrinogen is greater than 200 mg/mL, difficulty of dissolution occurs and it is difficult to use it due to very high viscosity. Therefore, it is preferred to use fibrinogen within the range of 10 mg/mL to 200 mg/mL.

In addition, the concentration of the white lyophilized powder (thrombin) used herein is preferably in the range of 1 IU/mL to 200 IU/mL. Where the concentration of thrombin is less than 1 IU/mL, it is too low to form fibrin, thereby being incapable of achieving gelation within several minutes. In contrast, where the concentration of thrombin is greater than 200 IU/mL, thrombin is solidified immediately upon mixing and thereby is not suitable for use in the injectable therapeutic agent. Therefore, it is preferred to use thrombin within the range of 1 IU/mL to 200 IU/mL.

Additionally, the concentration of aprotinin used herein is preferably in the range of 100 KIU/mL to 20,000 KIU/mL. Where the concentration of aprotinin is less than 100 KIU/mL, it is of little help to inhibition of fibrin decomposition. In contrast, where the concentration of aprotinin is greater than 20,000 KIU/mL, cytotoxicity may occur. Therefore, it is preferred to use aprotinin within the range of 100 KIU/mL to 20,000 KIU/mL.

Further, the amount of hyaluronic acid used herein is preferably in the range of 0.01% to 2% (w/v). Where the amount of hyaluronic acid is less than 0.01%, it is difficult to sufficiently exert functions thereof as the matrix. In contrast, where the amount of hyaluronic acid is greater than 2%, bindabilily of fibrin may be structurally affected, thereby affecting strength and hardness of the matrix. Therefore, it is preferred to use hyaluronic acid within the range of 0.01 to 2% (w/v).

Still further, the amount of collagen type 2 used herein is preferably in the range of 50 µg/mL to 1 mg/mL. Where the amount of collagen type 2 is less than 50 µg/mL it is difficult to sufficiently exert functions thereof. In contrast, where the amount of collagen type 2 is greater than 1 mg/mL, characteristics thereof affect bindability of fibrin, thereby affecting strength and hardness of the matrix. Therefore, it is preferred to use collagen type 2 within the range of 50 µg/mL to 1 mg/mL.

### Industrial Applicability

As apparent from the foregoing, the present invention provides the following effects: solution of inconvenience exhibited by conventional osteoperiosteal grafting, by mixing and transplanting matrices including fibrin, hyaluronic acid and collagen which are main ingredients of cartilage; in particular, convenient in situ transplantation due to rapid gelling characteristics in which an injectable cartilage therapeutic is gelated within a short period of time, i.e., about 1 to 5 minutes, upon transplanting; complete binding of transplanted sites to the adjacent native cartilages, as well as treatment of a wide variety of cartilage defects and severe osteoarthritis; alleviation of burden of patients to be treated via a simplified surgical operation method; and thereby promotion of rapider and more effective cartilage generation, resulting in increased customer satisfaction.

## Claims

1. A cartilage therapeutic composition for use in a method for transplanting an injectable chondrocyte for autologous chondrocyte transplantation, obtainable by:
placing 1 ml of a chondrocyte culture contained in Vial No. 1 (Red) into a 1 ml sterile syringe and injecting the cell culture into Vial No. 2 (Blue) containing white or light-yellow lyophilized powder fibrinogen by inserting the syringe into Vial No. 2 in the vertical direction, such that the concentration of fibrinogen is in the range of 10 mg/ml to 200 mg/ml;
injecting 1 ml of a cell culture contained in Vial No. 1 (Red) into Vial No. 3 (Red, small) containing white lyophilized powder thrombin by inserting the syringe into Vial No. 3 in the vertical direction, thereby gradually injecting red liquid;
collecting 0.1 cc of 1 ml content of Vial No. 3 (Red, small) using a 1 ml syringe,
followed by injection into a bottom of Vial No. 4 (Yellow), such that the concentration of thrombin is in the range of 1 IU/ml to 200 IU/ml;
adding the entire contents of two vials containing chondrocyte suspension to Vial No. 4 (Yellow) using a 1 ml sterile syringe, followed by mixing the contents two or three times using the syringe;
confirming complete dissolution of the contents of Vial No. 2 (Blue) and then injecting the entirety of the dissolved material into a 1 ml syringe;
injecting the well-mixed cell therapeutic of Vial 4 (Yellow) into a 1 ml syringe; and
mounting two 1 ml syringes filled with the contents of Vial No. 2 (Blue) and Vial No. 4 (Yellow) on a standing holder followed by mounting a screw tip on the syringes, and mixing the contents of the syringes using the front end mixing tip thereof wherein the resulting mixture is to be transplanted into a damaged cartilage region of an animal.

2. The composition according to claim 1, wherein the injecting step via insertion of a syringe needle is carried out by directly injecting the contents of the syringe over the lyophilizate, not injecting along the inner wall of the vial, while carefully preventing a needle tip from contacting the lyophilizate.

## Patentansprüche

1. Therapeutische Knorpelzusammensetzung zur Verwendung in einem Verfahren zur Transplantation eines injizierbaren Chondrozyten zur autologen Chondrozytentransplantation, erhältlich durch:
Aufnehmen von 1 ml einer Chondrozytenkultur aus Fläschchen Nr. 1 (Rot) in eine sterile 1 ml Spritze und Injizieren der Zellkultur in Fläschchen Nr. 2 (Blau), welches weißes oder hellgelbes lyophilisiertes Fibrinogenpulver enthält, indem die Spritze in vertikaler Richtung in Fläschchen Nr. 2 eingeführt wird, so dass die Konzentration des Fibrinogens im Bereich von 10 mg/ml bis 200 mg/ml beträgt,
Injizieren von 1 ml einer Zellkultur aus Fläschchen Nr. 1 (Rot) in Fläschchen Nr. 3 (Rot, klein), welches weißes lyophilisiertes Thrombinpulver enthält, indem die Spritze in vertikaler Richtung in Fläschchen Nr. 3 eingeführt wird, wodurch sukzessive rote Flüssigkeit injiziert wird,
Sammeln von 0.1 cm3 des Inhalts aus Fläschchen Nr. 3 (Rot, klein) unter Verwendung einer 1 ml Spritze,
wonach auf den Boden des Fläschchens Nr. 4 (Gelb) derart injiziert wird, dass die Konzentration des Thrombins im Bereich von 1 IU/ml bis 200 IU/ml beträgt,
Zugabe der gesamten Inhalte von zwei Chondrozytensuspension-enthaltenden Fläschchen unter Verwendung einer 1 ml Spritze in Fläschchen Nr. 4 (gelb) und
anschließendem zwei- oder dreimaligem Vermischen der Inhalte unter Verwendung der Spritze,
Feststellen, dass der Inhalt des Fläschchens Nr. 2 (Blau) vollständig gelöst ist und anschließendes Injizieren des gesamten gelösten Materials in eine 1 ml Spritze,
Injizieren des gut vermischten Zelltherapeutikums von Fläschchen 4 (Gelb) in eine 1 ml Spritze und
Befestigen von zwei mit dem Inhalt von Fläschchen Nr. 2 (Blau) und Fläschchen Nr. 4 (Gelb) gefüllten 1 ml Spritzen an einer Standhalterung und anschließendem Befestigen einer Schneckenspitze an die Spritzen und Vermischen der Inhalte der Spritzen unter Verwendung der vorderen Mischspitzen, wobei die erhaltene Mischung in eine geschädigte Knorpelregion eines Tieres transplantiert wird.

2. Zusammensetzung gemäß Anspruch 1, wobei der Schritt des Injizierens durch Einführung einer Kanüle durch direkte Injektion des Inhalts der Spritze über das Lyophilisat durchgeführt wird, wobei nicht über die innere Wand des Fläschchens injiziert wird, während sorgfältig verhindert wird, dass die Kanülenspitze mit dem Lyophilisat in Berührung kommt.

## Revendications

1. Composition thérapeutique de cartilage destinée à être utilisée dans un procédé de transplantation d'un chondrocyte injectable pour la transplantation de chondrocyte autologue, qui peut être obtenue par :
le transfert de 1 mL d'une culture de chondrocytes contenue dans le flacon n° 1 (rouge) dans une seringue stérile de 1 mL et l'injection de la culture cellulaire dans le flacon n° 2 (bleu) contenant du fibrinogène en poudre lyophilisée blanche ou jaune pâle par insertion de la seringue dans le flacon n°2 dans la direction verticale, de manière à ce que la concentration de fibrinogène se trouve dans la plage de 10 mg/mL à 200 mg/mL ;
l'injection de 1 mL d'une culture cellulaire contenue dans le flacon n° 1 (rouge) dans le flacon n° 3 (rouge, petit) contenant de la thrombine en poudre lyophilisée blanche par insertion de la seringue dans le flacon n° 3 dans la direction verticale, injectant ainsi progressivement le liquide rouge ;
la collecte de 0,1 cm³ d'un contenu de 1 mL du flacon n° 3 (rouge, petit) en utilisant une seringue de 1 mL, puis l'injection dans un fond du flacon n° 4 (jaune), de manière à ce que la concentration de thrombine se trouve dans la plage de 1 UI/mL à 200 UI/mL ;
l'ajout de la totalité des contenus des deux flacons contenant la suspension de chondrocytes au flacon n° 4 (jaune) en utilisant une seringue stérile de 1 mL, suivi par le mélange du contenu deux ou trois fois à l'aide de la seringue ;
la confirmation de la dissolution complète du contenu du flacon n° 2 (bleu) puis l'injection de la totalité de la substance dissoute dans une seringue de 1 mL ;
l'injection du produit thérapeutique cellulaire bien mélangé du flacon 4 (jaune) dans une seringue de 1 mL ; et
le montage de deux seringues de 1 mL remplies des contenus du flacon n° 2 (bleu) et du flacon n° 4 (jaune) sur un support suivi par le montage d'un embout de vis sur les seringues, et le mélange des contenus des seringues à l'aide de l'embout mélangeur avant de celles-ci, le mélange résultant étant destiné à être transplanté dans une région de cartilage endommagé d'un animal.

2. Composition selon la revendication 1, l'étape d'injection via l'insertion d'une aiguille de seringue étant réalisée en injectant directement les contenus de la seringue sur le lyophilisat, sans injecter le long de la paroi intérieure du flacon, tout en prenant soin d'empêcher le contact entre une pointe d'aiguille et le lyophilisat.
